# EUROPÄISCHE PATENTSCHRIFT

(11) **EP 0 972 882 B1**
(45) Veröffentlichungstag und Bekanntmachung des Hinweises auf die Patenterteilung: **03.12.2003**
(21) Anmeldenummer: 99106909.7
(22) Anmeldetag: 08.04.1999
(51) Int. Cl.: D21G 9/00, G01N 33/34, G01N 21/86

(54) **Messsystem**
Measuring system
Système de mesure

(30) Priorität: 14.07.1998 DE 19831612
(43) Veröffentlichungstag der Anmeldung: 19.01.2000
(73) Patentinhaber: Voith Paper Patent GmbH, 89522 Heidenheim (DE)
(72) Erfinder: Meinecke, Albrecht Dr., 89520 Heidenheim (DE); Münch, Rudolf, 89551 Königsbronn (DE)

(56) Entgegenhaltungen:
- EP-A- 0 390 623
- EP-A- 0 731 350
- US-A- 4 786 817
- US-A- 5 071 514
- US-A- 5 073 712
- US-A- 5 357 335
- US-A- 5 365 084
- US-A- 5 715 158

## Beschreibung

Die Erfindung betrifft ein Messsystem zur Messung des Querprofils bestimmter Eigenschaften einer Materialbahn wie insbesondere einer Faserstoffbahn in einer Papier- und/oder Kartonmaschine oder einer Streichmaschine, mit wenigstens einer stationären Querprofilmessvorrichtung mit zumindest einer Strahlungsquelle zur Bestrahlung der Materialbahn, wenigstens einem Sensor zur Messung der Intensität einer durch die Materialbahn beeinflussten Strahlung, und wenigstens einer Mess- und/oder Auswerteelektronik. Sie betrifft ferner eine Papier- und/oder Kartonmaschine oder Streichmaschine mit einem solchen Messsystem. Ein Messsystem der eingangs genannten Art ist aus der US-A-4 786 817 bekannt.

Die üblichen Messsysteme zur Messung des Querprofils bestimmter Eigenschaften einer Materialbahn arbeiten in der Regel auf der Basis von IR-, α-, β-, γ-Strahlen mit traversierenden Messanordnungen. Aus "Wochenblatt für Papierfabrikation", Heft 11/12, 1998, S. 609 und 611 sind auch bereits nicht traversierende Messgeräte bekannt, die für eine spektroskopische Messung unmittelbar hinter dem Stoffauflauf einer Papiermaschine ausgelegt sind.

Die bisher bekannten Messsysteme weisen u.a. den Nachteil auf, dass die betreffende Messung überwiegend nur in langen, freien Zügen der Materialbahn möglich ist. Bei den herkömmlichen traversierenden Messsystemen ist eine reine Querprofilmessung praktisch ausgeschlossen, nachdem das Traversieren eine Art Zickzack-Abtastung mit sich bringt. Nachdem die Messung normalerweise nur in der Siebpartie und am Ende einer jeweiligen Papiermaschine möglich ist, kommt es angesichts der dazwischenliegenden Vielzahl von Stellgliedern in der Regel zu langen Laufzeiten, was eine relativ ungenaue Regelung mit sich bringt.

Zur Messung des Flächengewichtes ist auch bereits die Verwendung von CCD-Kameras bekannt. Durch diese kann zwar die gesamte produzierte Papierfläche erfasst werden. Von Nachteil ist jedoch insbesondere die aufwendige Bildverarbeitung.

Es sind auch bereits Messgeräte zur Messung der Leitfähgkeit des Stoffes auf dem Sieb im Nassteil bekannt geworden. Es solches Messgerät kann zwar relativ nahe an den betreffenden Stoffauflauf-Stellgliedern angeordnet werden. Ein Nachteil besteht jedoch darin, dass die Leitfähigkeit ein Maß für die Feuchtigkeit und nicht für die Flächenbezogene Masse ist. Eine Anwendung z.B. für Coater ist somit ausgeschlossen.

Bei dem aus der US-A-4 786 817 bekannten Messsystem ist als Strahlungsquelle eine Glühlampe vorgesehen, deren Licht zudem über eine Anordnung einer Vielzahl von Spiegeln quer über die Materialbahn verteilt wird. Die Messung erfolgt nach dem Transmissions- oder Durchlichtverfahren. Die Messung ist also nur im Bereich freier Bahnzüge möglich. Während die Glühlampe ein relativ breitbandiges Licht abgibt, sind den Sensoren verschiedene Bandpassfilter vorgeschaltet.

Auch in der US-A-5 073 712 ist wieder ein optisches Messsystem zur Messung von Eigenschaftsquerprofilen einer bewegten Materialbahn beschrieben. Dieses bekannte Messsystem weist einen Scanner auf, der wieder eine Glühlampe sowie ein Spiegelsystem umfasst.

In der EP-A-0 390 623 wird ein der Messung von Eigenschaftsquerprofilen einer bewegten Materialbahn dienendes optisches Messsystem beschrieben, bei dem das Licht der auch hier wieder durch eine Glühlampe gebildeten Lichtquelle über ein Glasfasernetz quer über die Materialbahn verteilt wird.

In der US-A-5 071 514 ist ein optisches Messsystem zur Messung von Eigenschaftsquerprofilen einer bewegten Materialbahn beschrieben, bei dem als Lichtquellen Halogenbirnen eingesetzt werden.

In der US-A-5 715 158 ist ein Messsystem für eine Papiermaschine beschrieben, das mehrere in Bahnlaufrichtung über die Maschine verteilte Sensoren umfasst, bei denen es sich um Scanner oder um stationäre Sensoren handeln kann. Die betreffenden Sensoren werden in dieser Druckschrift nicht näher beschrieben.

Die US-A-5 365 084 ist mit der Videoüberwachung bewegter Materialbahnen und der dafür erforderlichen Bahnbeleuchtung befasst. Um insbesondere hinsichtlich des Beleuchtungswinkels, der Lichtintensität, des Licht-Reinheitsgrades und des Lichtspektrums eine Anpassung an die jeweiligen Messbedingungen und hierbei insbesondere an die Beschaffenheit, zum Beispiel Farbe, der jeweiligen Materialbahn zu ermöglichen, umfasst das betreffende Beleuchtungssystem eine Anordnung von LEDs von teilweise unterschiedlicher spektraler Charakteristik. Für eine jeweilige optimale Anpassung sind die LEDs einzeln oder gruppenweise entsprechend ansteuerbar. Dabei wird ein jeweiliger Querstreifen der Bahn mehrfach abgetastet, wobei die jeweiligen Messwerte integriert werden.

Ziel der Erfindung ist es, ein verbessertes Messsystem der eingangs genannten Art zu schaffen, bei dem die zuvor angeführten Nachteile beseitigt und insgesamt kürzere Regelzeiten sowie eine genauere Regelung gewährleistet sind.

Diese Aufgabe wird nach der Erfindung dadurch gelöst, dass wenigstens eine stationäre Querprofilmessvorrichtung mit mehreren Leuchtdioden unterschiedlicher Wellenlängenbereiche als Strahlungsquellen versehen ist, um die Materialbahn in mehreren definierten unterschiedlichen Wellenlängenbereichen zu bestrahlen, und dass über einen jeweiligen Sensor zu einem bestimmten Zeitpunkt lediglich einer der durch die Leuchtdioden definierten unterschiedlichen Wellenlängenbereiche der Strahlung erfasst wird.

Aufgrund der erfindungsgemäßen Ausbildung ist eine genauere Messung des Querprofils bestimmter Eigenschaften bei verringertem Platzbedarf und erhöhter Wirtschaftlichkeit möglich. Die Messung ist insbesondere auch an relativ unzugänglichen Stellen und sogar in Bereichen geschlossener Führung möglich, in denen die betreffende Materialbahn beispielsweise durch eine Walze, ein Band, ein Sieb und/oder einen Filz gestützt ist. Dazu kann beispielsweise mittels wenigstens eines Sensors eine von der Materialbahn oder deren Belag reflektierte Strahlung erfasst werden. Zusätzlich oder alternativ ist es auch möglich, mittels wenigstens eines Sensors eine durch das Materialband oder deren Belag hindurch getretene Strahlung zu erfassen. Die Erfassung einer von der Materialbahn bzw. deren Belag reflektierten Strahlung bringt zudem den Vorteil eines besonders geringen Platzbedarfs mit sich.

Der Einsatz von Leuchtdioden als Strahlungsquellen ist insbesondere angesichts der Langlebigkeit sowie der geringen Kosten solcher Bauelemente von Vorteil. Damit wird insbesondere auch ein seit langem bestehendes Vorurteil überwunden, wonach solche Leuchtdioden angeblich nicht geeignet sind.

In einem Wellenlängenbereich von 1300 bis 2400 nm gibt es im Absorptionsspektrum zwar relativ starke Peaks wie beispielsweise 1450 nm Wasser-Oberton, 1930 nm Wasser, 2100 nm Cellulosefaser, 2010 nm Clay, ca. 2300 nm Latex und Lignin, 2300 bis 2400 nm Polyethylen und andere Kunststoffe, wofür Leuchtdioden nicht verfügbar sind, so dass bisher Lampen mit weißglühenden Glühfäden eingesetzt werden. Untersuchungen haben jedoch gezeigt, dass durchaus auch mit mehreren LEDs anderer Wellenlängen beispielsweise auf die Flächenbezogenen Masse und erforderlichenfalls weitere Bahneigenschaften geschlossen werden kann. So ist die relative Messgenauigkeit, d.h. die Ansprechempfindlichkeit eines jeweiligen Sensors auf sehr geringe Eigenschaftsschwankungen sehr hoch. Die im Vergleich zu Lampen mit Glühfäden höhere Lebensdauer der LEDs ist insbesondere für die Anwendung der hier in Rede stehenden Querprofilmessung entscheidend, da hier üblicherweise 50 bis 500 Sensoren gleichzeitig eingesetzt werden und es für den Betreiber nicht zumutbar ist, laufend mit Lampen-Ausfällen rechnen zu müssen.

Auch hier ergibt sich ein äußerst geringer Platzbedarf insbesondere bei einer Beschränkung auf Rückstreulicht. Ideale Anwendungen in diesem Fall sind beispielsweise:
- Faserbelag auf dem Sieb im Nassteil
- Papier auf einem Filz in der Presse, einem Trockenfilz oder einem Trockensieb
- Papier auf einer Metallfläche (z.B. Walze)
- Strich-Belag auf einer Farb-Auftragswalze (z.B. Speedcoater)
- Strich auf einer Papierbahn

Alternativ oder zusätzlich ist grundsätzlich auch eine Messung im Durchlichtverfahren möglich, wobei dies in der Regel jedoch mit einem höheren Kostenaufwand verbunden ist.

Gemäß einer bevorzugten Ausführungsform des erfindungsgemäßen Messsystems sind wenigstens zwei in Bahnlaufrichtung einen Abstand voneinander aufweisende stationäre Querprofilmessungen vorgesehen.

Es können Filtermittel vorgesehen, um bestimmte Störgrößen und/oder den Einfluss wenigstens eines Aktuators bzw. Stellgliedes auf das jeweilige Querprofil herauszufiltern.

Insbesondere hinsichtlich eines möglichst geringen Platzbedarfs sowie einer erhöhten Wirtschaftlichkeit ist es zweckmäßig, wenn die Strahlungsquelle und/oder der Sensor an wenigstens einen Lichtleiter angeschlossen ist.

Bei einer zweckmäßigen Ausführungsform des erfindungsgemäßen Messsystems ist wenigstens eine stationäre Querprofilmessvorrichtung für einen Betrieb im Nahe-Infra-Rot-Bereich (NIR) ausgelegt ist.

Wenigstens eine stationäre Querprofilmessung kann beispielsweise zur quantitativen Erfassung des Flächengewichts, der Feuchte, der Dicke, bestimmter Inhaltsstoffe und/oder weiterer Eigenschaften der Materialbahn ausgelegt sein.

Insbesondere für eine Messung in Bereichen eines freien Bahnzuges kann eine jeweilige stationäre Querprofilmessung zweckmäßigerweise mit zumindest einer optischen Strahlungsquelle versehen sein, um die Materialbahn zu bestrahlen, und wenigstens einen Photoempfänger umfassen, um die Intensität der durch die Materialbahn beeinflussten optischen Strahlung zu messen.

Wird die Materialbahn mittels mehrerer Strahlungsquellen unterschiedlicher Wellenlängenbereiche bestrahlt, so kann die Materialbahn beispielsweise zeitlich nacheinander mittels der einzelnen unterschiedlichen Strahlungsquellen und/oder mittels unterschiedlicher Kombinationen von Strahlungsquellen bestrahlt werden.

Bei einer weiteren vorteilhaften Ausführungsform des erfindungsgemäßen Messsystems ist wenigstens eine stationäre Querprofilmessvorrichtung mit mehreren Sensoren unterschiedlicher spektraler Empfindlichkeit versehen, während die jeweilige stationäre Querprofilmessvorrichtung vorzugsweise lediglich eine Strahlungsquelle umfasst, um die Materialbahn zu bestrahlen.

Grundsätzlich ist es möglich, wenigstens eine Sensor/Filter-Einheit vorzusehen, der spektrale Empfindlichkeit und/oder Durchlässigkeit einstellbar ist.

Bei einer bevorzugten praktischen Ausführungsform ist wenigstens eine stationäre Querprofilmessvorrichtung mit Mitteln versehen, um eine durch die Materialbahn beeinflusste optische Strahlung sensorseitig spektral aufzuteilen. Dabei kann die betreffende stationäre Querprofilmessvorrichtung ein von der aufgeteilten Strahlung beaufschlagbares Photodiodenarray mit beispielsweise wenigstens 16 und insbesondere 265 Sensoren umfassen.

Die Strahlungsquellen können in einer gemeinsamen Umschließung vorgesehen sein und demzufolge nach außen hin wie eine einzige Strahlungsquelle in Erscheinung treten, bei der abhängig von elektrischen Eingängen die Art der ausgesendeten Strahlung verändert werden kann. So ist beispielsweise eine Lampe mit mehreren verschiedenen Glühfäden denkbar.

Die Mess- und/oder Auswerteelektronik umfasst vorteilhafterweise Mittel, um die Materialbahn über wenigstens eine stationäre Querprofilmessvorrichtung zeitlich nacheinander mittels einzelner Strahlungsquellen unterschiedlicher Wellenlängenbereiche und/oder mittels unterschiedlicher Kombinationen von Strahlungsquellen zu bestrahlen.

Ein zusätzlicher Rückschluss auf die Absorption bei verschiedenen Wellenlängen ist insbesondere dann möglich, wenn wenigstens zwei in unterschiedlicher Entfernung zu den Strahlungsquellen angeordnete Sensoren verwendet werden.

Grundsätzlich ist es auch möglich, dass den Strahlungsquellen wenigstens einer stationären Querprofilmessvorrichtung jeweils zumindest ein eigener Sensor oder ein eigenes Sensorpaar zugeordnet ist. Hierbei ist der Platzaufwand zwar etwas höher. Von Vorteil ist jedoch, dass die über die Sensoren erhaltenen Messergebnisse gleichzeitig abgefragt werden können.

Bei einer bevorzugten praktischen Ausführungsform des erfindungsgemäßen Messsystems umfasst wenigstens eine stationäre Querprofilmessvorrichtung vorzugsweise für jeden Messpunkt jeweils drei optische Strahlungsquellen und wenigstens einen Sensor. Damit stehen jeweils zumindest drei Signale zur Verfügung. Die benötigte Hardware lässt sich einfach integrieren. Sie benötigt wenig Platz an der Maschine und ist sehr preisgünstig herstellbar.

Gemäß einer vorteilhaften praktischen Ausführungsform umfasst wenigstens eine stationäre Querprofilmessvorrichtung vorzugsweise für jeden Messpunkt wenigstens eine Infrarot-Leuchtdiode (z.B. 880 nm oder 950 nm), eine Rot-Leuchtdiode (z.B. 635 nm) und eine Blau-Leuchtiode (z.B. 480 nm, eventuell Gallium-Nitrit-LED mit 430 nm).

Der Abstrahlwinkel der Leuchtdioden hat maßgeblichen Einfluss auf das Messverfahren. Vorzugsweise ist wenigstens eine stationäre Querprofilmessvorrichtung mit wenigstens einer Leuchtdiode versehen, deren Abtastwinkel in einem Bereich zwischen etwa 12° und etwa 30° liegt. Der Abstand zur Oberfläche bzw. dem Belag der Materialbahn liegt zweckmäßigerweise in einem Bereich von etwa 5 bis etwa 20 mm.

Die Schaltfrequenz der Leuchtdioden sollte im Vergleich mit dem Zeitraster, in dem die Eigenschafts-Messwerte bestätigt werden, hoch sein. Begrenzt wird sie durch die Grenzfrequenzen der Sensoren und der Leuchtdioden sowie durch die Geschwindigkeit der Auswerteelektronik. In der Praxis sind beispielsweise etwa 1000 Schaltvorgänge pro Sekunde oder mehr denkbar. Damit ist es möglich, beispielsweise alle 0,5 Sekunden einen Eigenschafts-Messwert zu erhalten, der selbst wiederum aus der Auswertung von mehr als 500 Einzelmessungen entstanden ist. Dasselbe Ergebnis kann beispielsweise auch dadurch erreicht werden, dass eine Schaltfrequenz von 50 Hertz gewählt wird und nach einem Umschaltvorgang 10 Einzelmesswerte genommen werden.

Aus den gewonnenen Messwerten kann beispielsweise durch einen Vergleich mit Erfahrungswerten auf die wichtigsten Eigenschaften der Materialbahn geschlossen werden. Es hat sich gezeigt, dass sich beispielsweise eine allgemeine Flächengewichtsänderung in allen Messsignalen etwa gleich auswirkt, während eine Änderung anderer Eigenschaften sehr unterschiedliche Auswirkungen auf diese Signale besitzt. Durch Experimente lässt sich prüfen, wie sich z.B. eine Änderung der Feuchte in den Messsignalen niederschlägt. Diese Kenntnisse können in einen Auswerte-Algorithmus eingebracht werden. In der Mess- und/oder Auswerteelektronik wird dieser Algorithmus dann beispielsweise dazu verwendet, aus den Messsignalen die verschiedenen Eigenschaften quantitativ zu bestimmen.

Bei der in der Praxis bevorzugten Ausführungsform des erfindungsgemäßen Messsystems ist die Mess- und/oder Auswerteelektronik gleichzeitig mehreren, vorzugsweise sämtlichen stationären Querprofilmessvorrichtungen zugeordnet.

Die erfindungsgemäße Papier- und/oder Kartonmaschine oder Streichmaschine ist dadurch gekennzeichnet, dass sie ein erfindungsgemäßes Messsystem umfasst.

Dabei können vorteilhafterweise wenigstens einer Einheit wie beispielsweise der Pressenpartie, der Trockenpartie usw. der Papier- und/oder Karton- bzw. Streichmaschine jeweils wenigstens zwei in Bahnlaufrichtung einen Abstand voneinander aufweisende stationäre Querprofilmessvorrichtungen zugeordnet sein.

Insbesondere im Hinblick auf kurze Regelzeiten ist es von Vorteil, wenn unmittelbar vor und/oder unmittelbar hinter wenigstens einem das jeweilige Querprofil beeinflussenden Aktuator oder Stellglied jeweils wenigstens eine stationäre Querprofilmessvorrichtung angeordnet ist.

Aufgrund der erfindungsgemäßen Ausbildung ist es somit insbesondere auch möglich, zumindest in der Pressenpartie und/oder Trockenpartie einer betreffenden Papier- und/oder Kartonmaschine jeweils wenigstens eine stationäre Querprofilmessvorrichtung vorzusehen.

Alternativ oder zusätzlich ist es auch möglich, dass zumindest in der Siebpartie und/oder am Ende einer Papier- und/oder Kartonmaschine wenigstens eine solche stationäre Querprofilmessvorrichtung vorgesehen ist.

Indem man einen jeweiligen Messort möglichst nahe an dem betreffenden Stellort wählt, kann einer betreffenden Messposition auf der Materialbahn bzw. deren Belag eindeutig unmittelbar ein im wesentlichen zuständiges Stellglied zugeordnet werden, so dass die Messstelle und der Aktuator bzw. das Stellglied über eine gemeinsame Elektronik eine schnelle Regelung gestatten. Wirken die Aktuatoren in erheblichem Maße in die Breite, so können sich die Aktuator-Elektroniken gegenseitig über ihre Verstellungen informieren, so dass jeder Aktuator bei einer Verstellung in Betracht ziehen kann, wie sich die anderen gerade verstellen.

Dadurch wird der Zeitverlust vermieden, der ansonsten zur Übertragung der Daten an einen sogenannten Messwerterfassungsrechner, von dort an einen sogenannten Querprofilrechner und von dort schließlich an das betreffende Stellglied bzw. den betreffenden Aktuator auftritt.

Beispielsweise im Zusammenhang mit einem sogenannten Speedcoater kann der zugrundeliegende Regelkreis die Schichtdicke auf der Auftragswalze konstant halten. Ein überlagerter Regelkreis sorgt beispielsweise für die korrekte Einhaltung von Absolutwerten des Strichauftrags.

In den Unteransprüchen sind weitere vorteilhafte Ausführungsvarianten des erfindungsgemäßen Meßsystems angegeben.

Die Erfindung wird im folgenden anhand von Ausführungsbeispielen und unter Bezugnahme auf die Zeichnungen näher erläutert; in dieser zeigen:
- Figur 1: in rein schematischer Darstellung eine erste Ausführungsform eines Meßsystems zur Messung des Querprofils bestimmter Eigenschaften einer Materialbahn und
- Figur 2: eine rein schematische Darstellung einer weiteren Ausführungsform des Meßsystems.

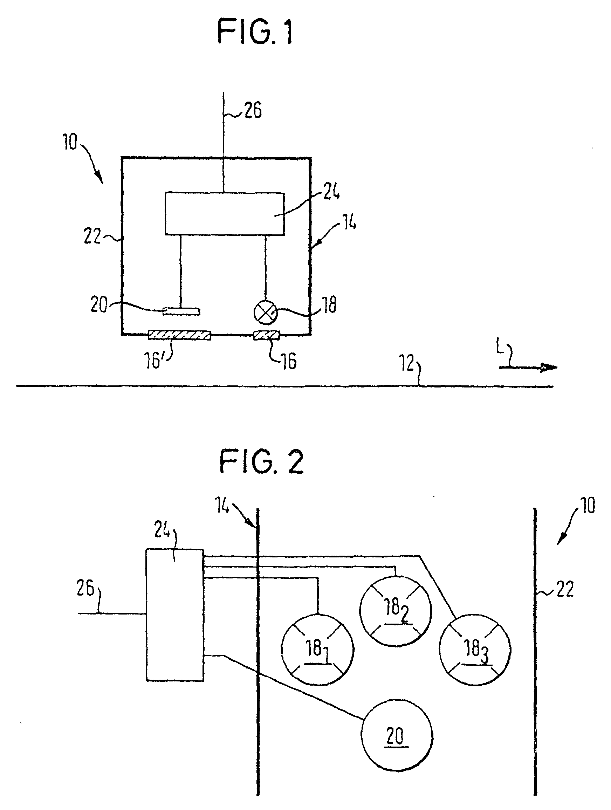

Figur 1 zeigt in rein schematischer Darstellung ein Meßsystem 10 zur Messung des Querprofils bestimmter Eigenschaften einer Materialbahn 12, insbesondere einer Faserstoffbahn, in einer Papier- und/oder Kartonmaschine oder einer Streichmaschine.

Das Meßsystem 10 umfaßt wenigstens eine stationäre Querprofilmeßvorrichtung 14 mit mehreren über die Arbeitsbreite verteilten Strahlungsquellen 18 und Sensoren 20 zur Messung der Intensität einer durch die Materialbahn 12 beeinflußten Strahlung. Dabei können für einen jeweiligen Meßpunkt auch mehrere Strahlungsquellen 18 und/ oder auch mehrere Sensoren 20 vorgesehen sein. Es können beispielsweise 50 Meßpunkte pro Meter vorgesehen sein. Grundsätzlich ist jedoch auch eine andere Meßpunktedichte möglich.

Die Materialbahn 12 wird in Richtung des Pfeils L unter der stationären Querprofilmeßvorrichtung 14 vorbeigeführt, wobei im Gehäuse 22 der stationären Querprofilmeßvorrichtung 14 Sichtfenster 16, 16' vorgesehen sind, denen für einen jeweiligen Meßpunkt jeweils wenigstens eine Leuchtiode bzw. wenigstens ein Sensor 20 wie insbesondere ein Photoempfänger gegenüberliegt, die in dem Gehäuse 22 der stationären Querprofilmeßvorrichtung 14 angeordnet sind. Die Sichtfenster 16, 16' sind im Abstand von der Materialbahn 12 angeordnet. Zweckmäßig ist es, wenn der Abstand insbesondere zwischen den Sensoren 20 und der Materialbahn 12 bzw. deren Belag in einem Bereich von etwa 5 bis etwa 20 mm liegt.

Der stationären Querprofilmeßvorrichtung 14 ist ferner eine Meßund/oder Auswerteelektronik 24 zugeordnet, die im vorliegenden Fall innerhalb des Gehäuses 22 angeordnet ist. Diese Meß- und/oder Auswerteelektronik 24 kann über wenigstens eine Leitung 26 an ein übergeordnetes System, insbesondere ein Prozeßleitsystem und/oder eine Spannungsversorgung, angeschlossen sein.

Die optische Bestrahlung der Materialbahn 12 bzw. deren Belag erfolgt in mehreren definierten unterschiedlichen Wellenlängenbereichen. Mittels der wenigstens einen einer jeweiligen Meßstelle zugeordneten Strahlungsquelle 18 wird die Intensität der durch die Materialbahn 12 bzw. deren Belag beeinflußten Strahlung gemessen. Dabei erfaßt jeder dem betreffenden Meßpunkt zugeordnete Sensor 20 zu einem bestimmten Zeitpunkt lediglich einen der definierten unterschiedlichen Wellenlängenbereiche der Strahlung.

Beim vorliegenden Ausführungsbeispiel wird lediglich die von der Materialbahn 12 bzw. deren Belag reflektierte Strahlung erfaßt, wodurch der erforderliche Platzbedarf auf ein Minimum herabgesetzt ist.

Zweckmäßigerweise sind wenigstens zwei in Bahnlaufrichtung L einen Abstand voneinander aufweisende stationäre Querprofilmeßvorrichtungen vorgesehen. So können solche stationäre Querprofilmeßvorrichtungen insbesondere auch in der Pressenpartie und/oder Trockenpartie der betreffenden Papier- und/oder Kartonmaschine vorgesehen sein. Dabei kann eine solche stationäre Querprofilmeßvorrichtung insbesondere auch in einem Bereich geschlossener Bahnführung angesetzt werden. Grundsätzlich ist es jedoch auch möglich, in der Siebpartie und/oder am Ende einer Papier- und/ oder Kartonmaschine eine solche stationäre Querprofilmeßvorrichtung vorzusehen. Zur Erzielung möglichst kurzer Regelzeiten kann eine jeweilige stationäre Querprofilmeßvorrichtung insbesondere unmittelbar vor und/oder unmittelbar hinter wenigstens einem das jeweilige Querprofil beeinflussenden Aktuator oder Stellglied angeordnet sein.

Figur 2 zeigt in rein schematischer Darstellung eine weitere Ausführungsform einer stationären Querprofilmeßvorrichtung 14 des Meßsystems 10, wobei lediglich die für einen Meßpunkt bestimmte Gruppe von Strahlungsquellen und Sensoren dargestellt ist.

Wie anhand der Figur 2 zu erkennen ist, enthält die wieder mit einem Gehäuse 22 versehene stationäre Querprofilmeßvorrichtung 14 für einen jeweiligen Meßpunkt drei optische Strahlungsquellen 18 und wenigstens einen Sensor bzw. Photoempfänger 20. Sowohl die Strahlungsquellen 18 als auch die Sensoren 20 sind jeweils an die Meß- und/oder Auswerteelektronik 24 angeschlossen, die wieder über wenigstens eine Leitung 26 mit einem übergeordneten System, insbesondere einem Prozeßleitsystem und/oder einer Spannungsversorgung verbunden sein kann. Im vorliegenden Fall ist die Meß- und/oder Auswerteelektronik 24 außerhalb des Gehäuses 22 angeordnet.

Die drei optischen Strahlungsquellen werden im vorliegenden Fall durch eine Infrarot-Leuchtdiode 18₁, eine Rot-Leuchtdiode 18₂ und eine Blau-Leuchtdiode 18₃ gebildet. Die Materialbahn 12 wird demzufolge durch drei Strahlungsquellen unterschiedlicher Wellenlängenbereiche bestrahlt.

Grundsätzlich ist es auch möglich, je Meßpunkt jeweils zwei Sensoren bzw. Photoempfänger 20 vorzusehen, die dann zweckmäßigerweise in unterschiedlicher Entfernung zu den Strahlungsquellen 18 anzuordnen sind.

Die Meß- und/oder Auswerteelektronik 24 steuert den zeitlichen Ablauf der Bestrahlung und Messung. Sie kann z.B. in (vgl. Figur 1) oder auf dem Gehäuse 22 angeordnet, oder wie im vorliegenden, in einem entfernten Gehäuse untergebracht sein.

Die Meß- und/oder Auswerteelektronik 24 oder ein betreffendes übergeordnetes System kann grundsätzlich wenigstens ein Signal liefern, das beispielsweise für das Flächengewicht, die Feuchte, die Dicke, bestimmte Inhaltastoffe und/oder weitere Eigenschaften der Materialbahn 12 bzw. des Belags dieser Materialbahn 12 repräsentativ ist.

### Bezugszeichenliste

- 10: Meßsystem
- 12: Materialbahn
- 14: stationäre Querprofilmeßvorrichtung
- 16: Sichtfenster
- 16': Sichtfenster
- 18: optische Strahlungsquelle / Leuchtdiode
- 18₁: Infrarot-Leuchtdiode
- 18₂: Rot-Leuchtdiode
- 18₃: Blau-Leuchtdiode
- 20: Sensor / Photoempfänger
- 22: Gehäuse
- 24: Meß- und/oder Auswerteelektronik
- 26: Leitung

- L: Bahnlaufrichtung

## Patentansprüche

1. Messsystem (10) zur Messung des Querprofils bestimmter Eigenschaften einer Materialbahn (12) wie insbesondere einer Faserstoffbahn in einer Papier- und/oder Kartonmaschine oder einer Streichmaschine, mit wenigstens einer stationären Querprofilmessvorrichtung (14) mit zumindest einer Strahlungsquelle (18) zur Bestrahlung der Materialbahn (12), wenigstens einem Sensor (20) zur Messung der Intensität einer durch die Materialbahn (12) beeinflussten Strahlung, und wenigstens einer Mess- und/oder Auswerteelektronik (24),
**dadurch gekennzeichnet,**
**dass** wenigstens eine stationäre Querprofilmeßvorrichtung (14) mit mehreren Leuchtdioden (18) unterschiedlicher Wellenlängenbereiche als Strahlungsquellen versehen ist, um die Materialbahn(12) in mehreren definierten unterschiedlichen Wellenlängenbereichen zu bestrahlen, und dass über einen jeweiligen Sensor (20) zu einem bestimmten Zeitpunkt lediglich einer der durch die Leuchtdioden (18) definierten unterschiedlichen Wellenlängenbereiche der Strahlung erfasst wird.

2. Messsystem nach Anspruch 1,
**dadurch gekennzeichnet ,**
**dass** wenigstens zwei in Bahnlaufrichtung (L) einen Abstand voneinander aufweisende stationäre Querprofilmessvorrichtungen (14) vorgesehen sind.

3. Messsystem nach einem der vorhergehenden Ansprüche,
**dadurch gekennzeichnet,**
**dass** Filtermittel vorgesehen sind, um bestimmte Störgrößen und/oder den Einfluss wenigstens eines Aktuators bzw. Stellgliedes auf das jeweilige Querprofil herauszufiltern.

4. Messsystem nach einem der vorhergehenden Ansprüche,
**dadurch gekennzeichnet,**
**dass** die Strahlungsquelle (18) und/oder der Sensor (20) an wenigstens einen Lichtleiter angeschlossen ist.

5. Messsystem nach einem der vorhergehenden Ansprüche,
**dadurch gekennzeichnet,**
**dass** wenigstens eine stationäre Querprofilmessvorrichtung (14) für einen Betrieb im Nahe-Infra-Rot-Bereich (NIR) ausgelegt ist.

6. Messsystem nach einem der vorhergehenden Ansprüche,
**dadurch gekennzeichnet,**
**dass** wenigstens eine stationäre Querprofilmessvorrichtung (14) zur quantitativen Erfassung des Flächengewichts, der Feuchte, der Dicke, bestimmter Inhaltsstoffe und/oder weiterer Eigenschaften der Materialbahn (12) ausgelegt ist.

7. Messsystem nach einem der vorhergehenden Ansprüche,
**dadurch gekennzeichnet,**
**dass** wenigstens eine stationäre Querprofilmessvorrichtung (14) mit zumindest einem Sensor (20) versehen ist, um eine durch die Materialbahn (12) und/oder deren Belag hindurchgetretene Strahlung zu erfassen.

8. Messsystem nach einem der vorhergehenden Ansprüche,
**dadurch gekennzeichnet,**
**dass** wenigstens eine stationäre Querprofilmessvorrichtung (14) mit zumindest einem Sensor (20) versehen ist, um eine durch die Materialbahn (12) und/oder deren Belag reflektierte Strahlung zu erfassen.

9. Messsystem nach einem der vorhergehenden Ansprüche,
**dadurch gekennzeichnet,**
**dass** wenigstens eine stationäre Querprofilmessvorrichtung (14) mit zumindest einer optischen Strahlungsquelle (18) versehen ist, um die Materialbahn (12) zu bestrahlen, und wenigstens einen Photoempfänger (20) umfasst, um die Intensität der durch die Materialbahn (12) beeinflussten optischen Strahlung zu messen.

10. Messsystem nach wenigstens einem der vorhergehenden Ansprüche, **dadurch gekennzeichnet,**
**dass** wenigstens eine stationäre Querprofilmessvorrichtung (14) mit mehreren Sensoren (20) unterschiedlicher spektraler Empfindlichkeit versehen ist, während sie vorzugsweise lediglich eine Strahlungsquelle (18) umfasst, um die Materialbahn (12) zu bestrahlen.

11. Messsystem nach einem der vorhergehenden Ansprüche,
**dadurch gekennzeichnet,**
**dass** wenigstens eine Sensor/Filter-Einheit vorgesehen ist, deren spektrale Empfindlichkeit und/oder Durchlässigkeit einstellbar ist.

12. Messsystem nach einem der vorhergehenden Ansprüche,
**dadurch gekennzeichnet,**
**dass** wenigstens eine stationäre Querprofilmessvorrichtung (14) mit Mitteln versehen ist, um eine durch die Materialbahn (12) beeinflusste optische Strahlung sensorseitig spektral aufzuteilen, und ein von der aufgeteilten Strahlung beaufschlagbares Photodiodenarray mit wenigstens 16 und insbesondere 265 Sensoren (20) umfasst.

13. Messsystem nach einem der vorhergehenden Ansprüche,
**dadurch gekennzeichnet ,**
**dass** die Mess- und/oder Auswerteelektronik (24) Mittel umfasst, um die Materialbahn (12) über wenigstens eine stationäre Querprofilmessvorrichtung (14) mit zeitlich nacheinander mittels einzelner Strahlungsquellen unterschiedlicher Wellenlängenbereiche oder/oder mittels unterschiedlicher Kombinationen von Strahlungsquellen zu bestrahlen.

14. Messsystem nach einem der vorhergehenden Ansprüche,
**dadurch gekennzeichnet ,**
**dass** wenigstens eine stationäre Querprofilmessvorrichtung (14) mit wenigstens zwei in unterschiedlicher Entfernung zu den Strahlungsquellen (18) angeordneten Sensoren (20) versehen ist.

15. Messsystem nach wenigstens einem der vorhergehenden Ansprüche,
**dadurch gekennzeichnet ,**
**dass** den Strahlungsquellen (18) wenigstens einer stationären Querprofilmessvorrichtung (14) jeweils zumindest ein eigener Sensor (20) oder ein eigenes Sensorpaar zugeordnet ist.

16. Messsystem nach wenigstens einem der vorhergehenden Ansprüche,
**dadurch gekennzeichnet,**
**dass** wenigstens eine stationäre Querprofilmessvorrichtung (14) vorzugsweise für jeden Messpunkt jeweils drei optische Strahlungsquellen (18) und wenigstens einen Sensoren (20) umfasst.

17. Messsystem nach wenigstens einem der vorhergehenden Ansprüche,
**dadurch gekennzeichnet ,**
**dass** wenigstens eine stationäre Querprofilmessvorrichtung (14) vorzugsweise für jeden Messpunkt wenigstens eine Infrarot-, wenigstens eine Rotund/oder wenigstens eine Blau-Leuchtdiode (18) umfasst.

18. Messsystem nach wenigstens einem der vorhergehenden Ansprüche,
**dadurch gekennzeichnet ,**
**dass** wenigstens eine stationäre Querprofilmessvorrichtung (14) mit wenigstens einer Leuchtdiode (18) versehen ist, deren Abstrahlwinkel in einem Bereich zwischen etwa 12° und etwa 30° liegt.

19. Messsystem nach einem der vorhergehenden Ansprüche,
**dadurch gekennzeichnet,**
**dass** die Mess- und/oder Auswerteelektronik (24) gleichzeitig mehreren stationären Querprofilmessvorrichtungen (14) zugeordnet ist.

20. Papier- und/oder Kartonmaschine oder Streichmaschine mit einem Messsystem (10) nach einem der vorhergehenden Ansprüche.

21. Maschine nach Anspruch 20,
**dadurch gekennzeichnet,**
**dass** wenigstens einer Einheit der Papier- und/oder Karton- bzw. Streichmaschine jeweils wenigstens zwei in Bahnlaufrichtung (L) einen Abstand voneinander aufweisende stationäre Querprofilmessvorrichtungen (14) zugeordnet sind.

22. Maschine nach Anspruch 20 oder 21,
**dadurch gekennzeichnet,**
**dass** unmittelbar vor und/oder unmittelbar hinter wenigstens einem das jeweilige Querprofil beeinflussenden Aktuator oder Stellglied jeweils eine stationäre Querprofilmessvorrichtung (14) angeordnet ist.

23. Maschine nach einem der Ansprüche 20 bis 22,
**dadurch gekennzeichnet,**
**dass** die Maschine eine Papier- und/oder Kartonmaschine ist , und
**dass** zumindest in der Pressenpartie und/oder Trockenpartie der Maschine jeweils wenigstens eine stationäre Querprofilmessvorrichtung (14) vorgesehen ist.

24. Maschine nach einem der Ansprüche 20 bis 23,
**dadurch gekennzeichnet,**
**dass** wenigstens eine stationäre Querprofilmessvorrichtung (14) in einem Bereich geschlossener Bahnführung vorgesehen ist.

25. Maschine nach einem der Ansprüche 20 bis 24,
**dadurch gekennzeichnet,**
**dass** die Maschine eine Papier- und/oder Kartonmaschine ist , und
**dass** zumindest in der Siebpartie und/oder am Ende der Maschine wenigstens eine stationäre Querprofilmessvorrichtung (14) vorgesehen ist.

## Claims

1. Measuring system (10) for measuring the cross-machine profile of specific properties of a material web (12), such as in particular a fibrous web in a paper and/or board machine or a coating machine, having at least one stationary cross-machine profile measuring apparatus (14) comprising at least one radiation source (18) for irradiating the material web (12), at least one sensor (20) for measuring the intensity of radiation affected by the material web (12), and at least one measuring and/or evaluation electronic unit (24),
**characterized in that**
at least one stationary cross-machine profile measuring apparatus (14) is provided with a plurality of light-emitting diodes (18) of different wavelength ranges as radiation sources, in order to irradiate the material web (12) in a plurality of defined different wavelength ranges, and **in that**, by means of a respective sensor (20), only one of the different wavelength ranges of the radiation defined by the light-emitting diodes (18) is registered at a specific time.

2. Measuring system according to Claim 1, **characterized in that** at least two stationary cross-machine profile measuring apparatuses (14) are provided at a distance from each other in the web running direction (L).

3. Measuring system according to one of the preceding claims, **characterized in that** filtering means are provided in order to filter out specific interfering variables and/or the influence of at least one actuator or actuating element on the respective cross-machine profile.

4. Measuring system according to one of the preceding claims, **characterized in that** the radiation source (18) and/or the sensor (20) are/is connected to at least one optical conductor.

5. Measuring system according to one of the preceding claims, **characterized in that** at least one stationary cross-machine profile measuring apparatus (14) is designed for operation in the near infrared range (NIR).

6. Measuring system according to one of the preceding claims, **characterized in that** at least one stationary cross-machine profile measuring apparatus (14) is designed for the quantitative registration of the grammage, the moisture, the thickness, specific contents and/or further properties of the material web (12).

7. Measuring system according to one of the preceding claims, **characterized in that** at least one stationary cross-machine profile measuring apparatus (14) is provided with at least one sensor (20) in order to register radiation that has passed through the material web (12) and/or its covering.

8. Measuring system according to one of the preceding claims, **characterized in that** at least one stationary cross-machine profile measuring apparatus (14) is provided with at least one sensor (20) in order to register radiation reflected by the material web (12) and/or its covering.

9. Measuring system according to one of the preceding claims, **characterized in that** at least one stationary cross-machine profile measuring apparatus (14) is provided with at least one optical radiation source (18) in order to irradiate the material web (12), and comprises at least one photoreceiver (20) in order to measure the intensity of the optical radiation affected by the material web (12).

10. Measuring system according to at least one of the preceding claims, **characterized in that** at least one stationary cross-machine profile measuring apparatus (14) is provided with a plurality of sensors (20) of different spectral sensitivity, while it preferably comprises only one radiation source (18) in order to irradiate the material web (12).

11. Measuring system according to one of the preceding claims, **characterized in that** at least one sensor/filter unit is provided, whose spectral sensitivity and/or transmissivity can be adjusted.

12. Measuring system according to one of the preceding claims, **characterized in that** at least one stationary cross-machine profile measuring apparatus (14) is provided with means to divide up spectrally on the sensor side optical radiation affected by the material web (12), and comprises a photodiode array to which the divided radiation can be applied, with at least 16 and in particular 265 sensors (20).

13. Measuring system according to one of the preceding claims, **characterized in that** the measuring and/or evaluation electronic unit (24) comprises means for irradiating the material web (12) via at least one stationary cross-machine profile measuring apparatus (14) chronologically successively by means of individual radiation sources of different wavelength ranges and and/or by means of different combinations of radiation sources.

14. Measuring system according to one of the preceding claims, **characterized in that** at least one stationary cross-machine profile measuring apparatus (14) is provided with at least two sensors (20) arranged at different distances from the radiation sources (18).

15. Measuring system according to at least one of the preceding claims, **characterized in that** the radiation sources (18) of at least one stationary cross-machine profile measuring apparatus (14) are in each case assigned at least one dedicated sensor (20) or a dedicated pair of sensors.

16. Measuring system according to at least one of the preceding claims, **characterized in that** at least one stationary cross-machine profile measuring apparatus (14) preferably for each measuring point in each case comprises three optical radiation sources (18) and at least one sensor (20).

17. Measuring system according to at least one of the preceding claims, **characterized in that** at least one stationary cross-machine profile measuring apparatus (14), preferably for each measuring point, comprises at least one infrared, at least one red and/or at least one blue light-emitting diode (18).

18. Measuring system according to at least one of the preceding claims, **characterized in that** at least one stationary cross-machine profile measuring apparatus (14) is provided with at least one light-emitting diode (18) whose radiation angle lies in the range between about 12° and about 30°.

19. Measuring system according to one of the preceding claims, **characterized in that** the measuring and/or evaluation electronic unit (24) is assigned simultaneously to a plurality of stationary cross-machine profile measuring apparatuses (14).

20. Paper and/or board machine or coating machine having a measuring system (10) according to one of the preceding claims.

21. Machine according to Claim 20, **characterized in that** at least one unit of the paper and/or board or coating machine is in each case assigned at least two stationary cross-machine profile measuring apparatuses (14), at a distance from one another in the web running direction (L).

22. Machine according to Claim 20 or 21, **characterized in that** in each case a stationary cross-machine profile measuring apparatus (14) is arranged immediately upstream and/or immediately downstream of at least one actuator or actuating element that affects the respective cross-machine profile.

23. Machine according to one of claims 20 to 22, **characterized in that** the machine is a paper and/or board machine, and **in that** in each case at least one stationary cross-machine profile measuring apparatus (14) is provided, at least in the press section and/or the drying section of the machine.

24. Machine according to one of claims 20 to 23, **characterized in that** at least one stationary cross-machine profile measuring apparatus (14) is provided in a region with self-contained web guidance.

25. Machine according to one of claims 20 to 24, **characterized in that** the machine is a paper and/or board machine, and **in that** at least one stationary cross-machine profile measuring apparatus (14) is provided, at least at the wet end and/or at the end of the machine.

## Revendications

1. Système de mesure (10) pour la mesure du profil transversal de certaines propriétés d'une bande de matériau (12), comme notamment une bande de matière fibreuse dans une machine à papier et/ou à carton ou une machine à enduire, comportant au moins un dispositif de mesure de profil transversal stationnaire (14) avec au moins une source de rayonnement (18) pour irradier la bande de matériau (12), au moins un capteur (20) pour mesurer l'intensité d'un rayonnement influencé par la bande de matériau (12) et au moins une électronique de mesure et/ou d'exploitation (24),
**caractérisé en ce que**
au moins un dispositif de mesure de profil transversal stationnaire (14) est équipé de plusieurs diodes luminescentes (18) à plages de longueurs d'ondes différentes comme sources de rayonnement afin d'irradier la bande de matériau (12) dans plusieurs plages de longueurs d'ondes différentes définies, et que simplement une des plages de longueurs d'ondes différentes définies par les diodes luminescentes (18) du rayonnement est détectée au moyen d'un capteur respectif (20) à un certain moment.

2. Système de mesure selon la revendication 1,
**caractérisé en ce que**
il est prévu au moins deux dispositifs de mesure de profil transversal stationnaires (14) situés à distance l'un de l'autre dans le sens de circulation de la bande (L).

3. Système de mesure selon l'une quelconque des revendications précédentes,
**caractérisé en ce que**
il est prévu des moyens de filtrage afin de filtrer certaines grandeurs perturbatrices et/ou l'influence d'au moins un module ou élément de réglage sur le profil transversal respectif.

4. Système de mesure selon l'une quelconque des revendications précédentes,
**caractérisé en ce que**
la source de rayonnement (18) et/ou le capteur (20) sont raccordés à au moins un conducteur de lumière.

5. Système de mesure selon l'une quelconque des revendications précédentes,
**caractérisé en ce que**
au moins un dispositif de mesure de profil transversal stationnaire (14) est conçu pour un fonctionnement dans la plage proche de l'infrarouge (PIR).

6. Système de mesure selon l'une quelconque des revendications précédentes,
**caractérisé en ce que**
au moins un dispositif de mesure de profil transversal stationnaire (14) est conçu pour la détection quantitative du grammage, de l'humidité, de l'épaisseur, de certaines substances contenues et/ou d'autres propriétés de la bande de matériau (12).

7. Système de mesure selon l'une quelconque des revendications précédentes,
**caractérisé en ce que**
au moins un dispositif de mesure de profil transversal stationnaire (14) est équipé d'au moins un capteur (20) afin de détecter un rayonnement ayant traversé la bande de matériau (12) et/ou son revêtement.

8. Système de mesure selon l'une quelconque des revendications précédentes,
**caractérisé en ce que**
au moins un dispositif de mesure de profil transversal stationnaire (14) est équipé d'au moins un capteur (20) pour détecter un rayonnement reflété par la bande de matériau (12) et/ou son revêtement.

9. Système de mesure selon l'une quelconque des revendications précédentes,
**caractérisé en ce que**
au moins un dispositif de mesure de profil transversal stationnaire (14) est équipé d'au moins une source de rayonnement optique (18) afin d'irradier la bande de matériau (12) et comprend au moins un photorécepteur (20) afin de mesurer l'intensité du rayonnement optique influencé par la bande de matériau (12).

10. Système de mesure selon au moins l'une quelconque des revendications précédentes,
**caractérisé en ce que**
au moins un dispositif de mesure de profil transversal stationnaire (14) est équipé de plusieurs capteurs (20) à sensibilité spectrale différente, tandis qu'il comprend de préférence simplement une source de rayonnement (18) pour irradier la bande de matériau (12).

11. Système de mesure selon l'une quelconque des revendications précédentes,
**caractérisé en ce que**
il est prévu au moins une unité capteur/filtre dont la sensibilité spectrale et/ou la perméabilité est réglable.

12. Système de mesure selon l'une quelconque des revendications précédentes,
**caractérisé en ce que**
au moins un dispositif de mesure de profil transversal stationnaire (14) est équipé de moyens pour répartir au niveau spectral un rayonnement optique influencé par la bande de matériau (12) du côté du capteur et comprend un panneau de photodiodes susceptible de recevoir le rayonnement réparti et comportant au moins 16 et notamment 265 capteurs (20).

13. Système de mesure selon l'une quelconque des revendications précédentes,
**caractérisé en ce que**
l'électronique de mesure et/ou d'exploitation (24) comprend des moyens pour irradier la bande de matériau (12) par l'intermédiaire d'au moins un dispositif de mesure de profil transversal stationnaire (14) successivement dans le temps au moyen de sources de rayonnement individuelles à plages de longueurs d'ondes différentes et/ou au moyen de combinaisons différentes de sources de rayonnement.

14. Système de mesure selon l'une quelconque des revendications précédentes,
**caractérisé en ce que**
au moins un dispositif de mesure de profil transversal stationnaire (14) est équipé d'au moins deux capteurs (20) disposés à une distance différente des sources de rayonnement (18).

15. Système de mesure selon au moins l'une quelconque des revendications précédentes,
**caractérisé en ce que**
est associé aux sources de rayonnement (18) d'au moins un dispositif de mesure de profil transversal stationnaire (14) respectivement au moins un propre capteur (20) ou une propre paire de capteurs.

16. Système de mesure selon au moins l'une quelconque des revendications précédentes,
**caractérisé en ce que**
au moins un dispositif de mesure de profil transversal stationnaire (14) comprend de préférence pour chaque point de mesure respectivement trois sources de rayonnement optiques (18) et au moins un capteur (20).

17. Système de mesure selon au moins l'une quelconque des revendications précédentes,
**caractérisé en ce que**
au moins un dispositif de mesure de profil transversal stationnaire (14) comprend de préférence pour chaque point de mesure au moins une diode luminescente infrarouge, au moins une rouge et/ou au moins une bleue (18).

18. Système de mesure selon au moins l'une quelconque des revendications précédentes,
**caractérisé en ce que**
au moins un dispositif de mesure de profil transversal stationnaire (14) est équipé d'au moins une diode luminescente (18) dont l'angle d'émission se situe dans une plage allant d'environ 12° à environ 30°.

19. Système de mesure selon l'une quelconque des revendications précédentes,
**caractérisé en ce que**
l'électronique de mesure et/ou d'exploitation (24) est associée en même temps à plusieurs dispositifs de mesure de profil transversal stationnaires (14).

20. Machine à papier et/ou à carton ou machine à enduire comportant un système de mesure (10) selon l'une quelconque des revendications précédentes.

21. Machine selon la revendication 20,
**caractérisée en ce que**
à au moins une unité de la machine à papier et/ou à carton ou de la machine à enduire sont associés respectivement au moins deux dispositifs de mesure de profil transversal stationnaires (14) situés à distance l'un de l'autre dans le sens de circulation de la bande (L).

22. Machine selon la revendication 20 ou 21,
**caractérisée en ce que**
est disposé directement devant et/ou directement derrière au moins un module ou élément de réglage influant sur le profil transversal respectif respectivement un dispositif de mesure de profil transversal stationnaire (14).

23. Machine selon l'une quelconque des revendications 20 à 22,
**caractérisée en ce que**
il est prévu, au moins dans la partie presse et/ou la partie séchage de la machine, respectivement au moins un dispositif de mesure de profil transversal stationnaire (14).

24. Machine selon l'une quelconque des revendications 20 à 23,
**caractérisée en ce que**
au moins un dispositif de mesure de profil transversal stationnaire (14) est prévu dans une zone de guidage fermé de la bande.

25. Machine selon l'une quelconque des revendications 20 à 24,
**caractérisée en ce que**
la machine est une machine à papier et/ou à carton et qu'au moins dans la partie tamis et/ou à l'extrémité de la machine, il est prévu au moins un dispositif de mesure de profil transversal stationnaire (14).
